(19) Europäisches Patentamt
European Patent Office
Office européen des brevets

(11) EP 3 424 561 B1

(12) EUROPEAN PATENT SPECIFICATION

(45) Date of publication and mention of the grant of the patent:
**09.10.2019 Bulletin 2019/41**

(21) Application number: **17769370.2**

(22) Date of filing: **16.03.2017**

(51) Int Cl.:
***A61K 41/00*** (2006.01) ***A61N 5/10*** (2006.01)
***C07F 5/02*** (2006.01) ***A61P 35/00*** (2006.01)

(86) International application number:
**PCT/CN2017/076946**

(87) International publication number:
**WO 2017/162093 (28.09.2017 Gazette 2017/39)**

(54) **BORON NEUTRON CAPTURE TREATING SYSTEM AND ALPHA-AMINO ACID-LIKE BORON TRIFLUORIDE COMPOUNDS FOR USE IN TREATING TUMORS**

BOR-NEUTRONENEINFANGTHERAPIESYSTEM UND ALPHA-AMINOSÄUREÄHNLICHE BORTRIFLUORID VERBINDUNGEN ZUR VERWENDUNG BEI DER BEHANDLUNG VON TUMOREN

SYSTÈME DE TRAITEMENT PAR CAPTURE DE NEUTRONS DE BORE ET DES COMPOSES DE TRIFLUORURE DE BORE DE TYPE ACIDE ALPHA-AMINÉ POUR L'UTILISATION DANS LE TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2016 CN 201610180136**
**25.03.2016 CN 201610180591**
**25.03.2016 CN 201620241984 U**

(43) Date of publication of application:
**09.01.2019 Bulletin 2019/02**

(73) Proprietor: **Neuboron Medtech Ltd.**
**Nanjing, Jiangsu 211112 (CN)**

(72) Inventors:
- **WANG, Zheng**
**Nanjing**
**Jiangsu 211112 (CN)**
- **LI, Shihong**
**Nanjing**
**Jiangsu 211112 (CN)**
- **LUO, Zhigang**
**Nanjing**
**Jiangsu 211112 (CN)**
- **LIU, Yuanhao**
**Nanjing**
**Jiangsu 211112 (CN)**

(74) Representative: **Sun, Yiming**
**HUASUN Patent- und Rechtsanwälte**
**Friedrichstraße 33**
**80801 München (DE)**

(56) References cited:
**WO-A1-2014/007831 WO-A1-2015/156385**
**CN-A- 1 154 073 CN-A- 104 548 388**
**JP-B2- 5 383 237 US-A1- 2013 225 861**

- **Z. LIU ET AL: "Boramino acid as a marker for amino acid transporters", SCIENCE, vol. 1, no. 8, 11 September 2015 (2015-09-11), pages e1500694-e1500694, XP055499243, US ISSN: 0036-8075, DOI: 10.1126/sciadv.1500694**
- **R. F. BARTH ET AL: "Boron Neutron Capture Therapy of Cancer: Current Status and Future Prospects", CLINICAL CANCER RESEARCH, vol. 11, no. 11, 1 June 2005 (2005-06-01), pages 3987-4002, XP055554703, US ISSN: 1078-0432, DOI: 10.1158/1078-0432.CCR-05-0035**
- **ZHOU, YONGMAO: "T he Boron Neutron Capture Therapy (BNCT) Status to Go into the New Era", ENGINEERING SCIENCE, 5 December 2012 (2012-12-05), XP009510135,**

EP 3 424 561 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE DISCLOSURE

One aspect of the present disclosure relates to a radioactive ray irradiation therapy system, in particular to a boron neutron capture therapy system. Another aspect of the present disclosure relates to the field of medicine, in particular to the field of tumor-related medicine, and more specifically, $\alpha$-amino acid-like boron trifluoride compound for use in tumor therapy.

### BACKGROUND OF THE DISCLOSURE

Along with the development of atomic science, radiation therapy, for example, cobalt 60, linear accelerator, electron beam and so on, has become one of the main means of cancer treatment. However, limited by the physical condition of the radioactive ray itself, conventional photon or electronic therapy brings injury to a large number of normal tissues in the beam pathway while killing tumor cells. In addition, due to the different levels of sensitivity of tumor cells to radioactive rays, traditional radiation therapy normally has poor therapeutic effects towards malignant tumors with higher radiation resistance (for example, glioblastoma multiforme, melanoma).

In order to reduce the radiation injury of normal tissues surrounding the tumor, the conception of target therapy in the chemotherapy is applied in radiation therapy now. In addition, towards tumor cells with high radiation resistance, radiation sources having high relative biological effectiveness (RBE) are also energetically developed at the moment, such as proton therapy, heavy particle therapy, neutron capture therapy and so on. Among those, neutron capture therapy, such as boron neutron capture therapy, is a combination of the two conceptions described above. By means of specific agglomeration of boron containing drugs towards tumor cells, in combination with precise neutron beam regulation, boron neutron capture therapy provides a cancer treatment option better than the traditional radioactive rays.

Boron neutron capture therapy (BNCT) uses the characteristic of boron ($^{10}B$) containing drugs which have high capture cross-section with thermal neutrons, and produces two heavy charged particles $^4He$ and $^7Li$, through $^{10}B(n,\alpha)^7Li$ neutron capture nuclear fission reaction as follows:

$$^{1}n + {}^{10}B \longrightarrow {}^{11}B^{*} \longrightarrow {}^{7}Li + {}^{4}He + 2.79\ MeV \qquad (6.1\ \%)$$

$$\longrightarrow {}^{7}Li^{*} + {}^{4}He + 2.31\ MeV \qquad (93.9\ \%)$$

$$\downarrow$$

$$^{7}Li + \gamma\text{-ray}\ (0.48\ MeV)$$

The average energy of those two charged particles is about 2.33MeV. The two charged particles have the characteristics of high Linear Energy Transfer (LET) and short range. The Linear Energy Transfer and the range of $\alpha$ particle are respectively 150 keV/$\mu$m, 8$\mu$m. The Linear Energy Transfer and the range of heavy charged particle $^7Li$ are respectively 175 keV/$\mu$m, 5$\mu$m. The total range of two particles is approximately equivalent to the size of one cell, thus the radiation damage caused by them against living organism can be limited to the level of cells. When the boron containing drug selectively agglomerates in tumor cells, and matches with a proper neutron source, then it is possible to achieve the aim of killing tumor cells locally without harming normal tissues too much.

The effect of boron neutron capture therapy depends on the concentration of boron containing drug and the number of thermal neutrons in the location of tumor cells, so it is also known as binary cancer therapy; it can thus be seen that, in addition to the development of neutron source, the development of boron containing drugs plays an important role in the research of boron neutron capture therapy.

Tumors, especially malignant tumors, are diseases that seriously endanger human health in the world today. The mortality rates of tumors are second only to cardiovascular diseases, ranking second in the mortality rate of various types of diseases. In recent years, the incidence rates have shown a clear upward trend. According to the current incidence of cancer, the number of new cancer patients in the world will reach 15 million each year. Although the exact mechanisms of cancer developments are still unclear, most cancer patients are likely to survive if they can diagnose the cancer early and take early surgery, radiation or chemotherapy (or a combination of these methods).

A promising new form of high LET radiation cancer therapy is boron neutron capture therapy (BNCT). BNCT is a novel dual-targeted radiation therapy based on selective accumulation of boron, known as boron-10 or $^{10}B$, in tumors, followed by irradiation of tumors with thermalized neutrons. Thermalized neutrons strike boron-10, leading to nuclear fission (decay reaction). Nuclear fission reactions cause highly localized release of energy in the form of linear energy transfer (energy density, LET) radiation, which can kill cells more efficiently than low LET radiation, such as X-rays

(higher relative biological effects).

In BNCT, when administered in a therapeutically effective amount, the boron-containing compound must be non-toxic or low toxic, and can selectively accumulate in tumor tissue. Although BPA has the advantage of low chemical toxicity, it accumulates in critical normal tissues at below-desired levels. In particular, the ratio of boron in the tumor relative to normal brain and tumor to blood is approximately 3:1. This low specificity limits the maximum dose of BPA to the tumor because the allowable dose for normal tissue is a limiting factor.

Therefore, there is a need to develop new compounds that have a longer retention time in tumors and selectively target and destroy tumor cells with minimal damage to normal tissues. α-Amino acids are the main components of proteins and are the most important amino acids in organisms. They play a very important role in the production of ATP and in the process of neurotransmission. In addition, α-amino acids are also key nutrients for the survival and proliferation of cancer cells. Substitution of -COOH in the α-amino acid with $-BF_3$ yields an α-amino acid-like boron trifluoride compound, which is an α-amino acid isoelectronic compound. Studies have shown that the pathway for the uptake of α-amino acid-like boron trifluoride by the cells is the same as α-amino acids, and they all adopt enzyme-mediated pathways, and both have the same transport protein. The α-amino acid-like boron trifluoride compounds have attracted our intense attention in the design of novel boron carrier compounds for BNCT, which have high stability, good targeting, and high enrichment in tumor cells. Compared to FDG, the uptake of these compounds by the inflammatory region is almost negligible. In addition, the α-amino acid-like boron trifluoride compounds are readily synthesized and are usually prepared by reacting the corresponding boronic acid ester with $KHF_2$ under acidic conditions.

Boramino acids, in particular Phe-BF3, Leu-BF3, ALA-BF3 and Pro-BF3 were previously described (see Liu Z. et al., Science Adv 2015: 1(8), el500694). A high specific accumulation in tumor tissue was demonstrated.

WO 2014/007831 A1 discloses p-Boronophenylalanine (BPA, **1**) is a boronated amino acid

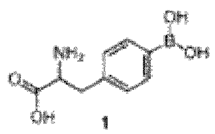

1

which exhibits a specific affinity for tumor cells.

Use of various derivatives of BPA and other boron-containing amino acids, such as glycine, alanine, aspartic acid, tyrosine, cysteine, and methionine, as well as non-naturally occurring amino acids was reported to be used in the boron neutron capture therapy (Barth R.F. et al., Clinical Cancer Research 2005: 11(11), 3987-4002).

In addition, using $^{18}$F-labeled α-amino acid-like boron trifluoride compounds in BNCT, boron concentrations and distributions in and around tumors and all tissues within the radiation therapy volume can be measured non-invasively, accurately and rapidly before and during irradiation. This diagnostic information enables boron neutron capture therapy to be performed faster, more accurately, and more safely by reducing the exposure of epithermal neutrons to tissue regions known to contain high levels of boron.

## SUMMARY

In order to achieve the improvement of the existing boron neutron capture therapy system, one aspect of the present disclosure provides a boron neutron capture therapy system including: a boron neutron capture therapy device and an α-amino acid-like boron trifluoride compound.

The α-amino acid-like boron trifluoride compound has a structure shown as formula (I):

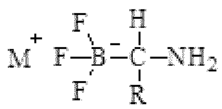

( I )

Wherein: R represents hydrogen, methyl, isopropyl, 1-methylpropyl, 2-methylpropyl, hydroxymethyl, 1-hydroxyethyl, benzyl or hydroxybenzyl; M represents H or metal atom.

The energy generated from the action of the neutron beam generated by the boron neutron capture therapy device on the α-amino acid-like boron trifluoride compound destroys tumor cell DNA.

BNCT is an ideal method for tumor therapy, which provides a new treatment for many tumors that cannot be treated with traditional methods.

Further, the tumor is a malignant tumor or metastatic tumor and is preferably glioma, recurrent head and neck

tumor, malignant melanoma, breast cancer or metastatic hepatoma. Malignant neoplasms are often referred to as cancers, and they are collectively referred to as more than 100 related diseases. When cells in the body are mutated, it will continue to divide and not be controlled by the body, eventually developed into cancer. Malignant tumor cells can invade and destroy adjacent tissues and organs, and the cells can pass through the tumor and enter the blood or lymphatic system. This is how malignant tumors form new tumors from the primary site to other organs. It is called metastasis of malignant tumors.

Furthermore, the tumor is a brain tumor or a melanoma. A brain tumor is a tumor that grows in the brain, including primary brain tumors that occur in the brain parenchyma and secondary brain tumors that metastasize from the rest of the body to the brain. Melanoma, also known as malignant melanoma, is a highly malignant tumor that produces melanin. It occurs in the skin or in the mucous membranes close to the skin. It is also found in the pia mater and the choroid.

Preferably, the brain tumor is glioma. Neuroepithelioma-derived tumors are called gliomas and account for 40-50% of brain tumors. They are common intracranial malignancies.

The α-amino acid-like boron trifluoride compound plays an important role in the application of the boron neutron capture therapy system, which will be described in details below.

Preferably, M in the α-amino acid-like boron trifluoride compound represents potassium or sodium.

Preferably, the element B in the α-amino acid-like boron trifluoride compound is $^{10}B$.

In order to further increase the $^{10}B$ content in the boron-containing drug, the purity of $^{10}B$ in the α-amino acid-like boron trifluoride compound is ≥ 95%.

At least one F atom of the α-amino acid-like boron trifluoride compound is $^{18}F$, such that boron concentrations and distributions in and around tumors and all tissues within the radiation therapy volume can be measured non-invasively, accurately and rapidly before and during irradiation. This diagnostic information enables boron neutron capture therapy to be performed faster, more accurately, and more safely by reducing the exposure of epithermal neutrons to tissue regions known to contain high levels of boron.

Further, the boron neutron capture therapy device includes a neutron generator and a beam shaping assembly for adjusting a neutron beam energy spectrum generated by the neutron generator to an epithermal neutron energy zone.

The beam shaping assembly also plays an important role in improving the flux and quality of neutron sources. The beam shaping assembly includes a moderator adjacent to the neutron generator, a reflector surrounding the moderator, a thermal neutron absorber adjacent to the moderator, and a radiation shield arranged in the beam shaping assembly, the neutron generator generates neutrons by a nuclear reaction with the incident proton beam, the moderator moderates neutrons generated from the neutron generator to an epithermal neutron energy zone, the reflector directs the deviated neutrons back to increase the intensity of the epithermal neutron beam, the thermal neutron absorber is used to absorb thermal neutrons to avoid overdosing in superficial normal tissues during therapy, and the radiation shield is used to shield leaking neutrons and photons to reduce dose of the normal tissue not exposed to irradiation.

The boron neutron capture therapy device further includes a collimator disposed at the beam outlet for converging the epithermal neutrons.

Another aspect of the present disclosure is to provide α-amino acid-like boron trifluoride compound having a structure shown as formula (I):

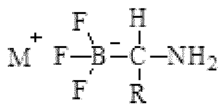

( I )

wherein: R represents hydrogen, methyl, isopropyl, 1-methylpropyl, 2-methylpropyl, hydroxymethyl, 1-hydroxyethyl, benzyl or hydroxybenzyl; M represents H or metal atom for use in tumor therapy.

The tumor therapy refers to boron neutron capture therapy of the tumor. The boron neutron capture therapy (BNCT) is a novel dual-targeted radiation therapy that destroys cancer cells through the nuclear fission of boron in the tumor cells. First of all, oral or intravenous injection of boron carrier agent with strong affinity for tumor cells, after the drug is enriched in tumor cells and irradiated with neutrons, the $^{10}B$ atom undergoes a nuclear fission reaction, generating α particles and $^{7}Li$ particles with high radiation energy and a small radiation range, which in turn selectively kills the tumor cells in which they are located. BNCT is an ideal method for tumor therapy, which provides a new therapy for many tumors that cannot be treated with traditional methods.

Further, the tumor is a malignant tumor or metastatic tumor and is preferably glioma, recurrent head and neck tumor, malignant melanoma, breast cancer or metastatic hepatoma. Malignant neoplasms are often referred to as cancers, and they are collectively referred to as more than 100 related diseases. When cells in the body are mutated, it will continue to divide and not be controlled by the body, eventually developed into cancer. Malignant tumor cells can

invade and destroy adjacent tissues and organs, and the cells can pass through the tumor and enter the blood or lymphatic system. This is how malignant tumors form new tumors from the primary site to other organs. It is called metastasis of malignant tumors.

Further, the tumor is a brain tumor or a melanoma. A brain tumor is a tumor that grows in the brain, including primary brain tumors that occur in the brain parenchyma and secondary brain tumors that metastasize from the rest of the body to the brain. Melanoma, also known as malignant melanoma, is a highly malignant tumor that produces melanin. It occurs in the skin or in the mucous membranes close to the skin. It is also found in the pia mater and the choroid.

Preferably, the brain tumor is glioma. Neuroepithelioma-derived tumors are called gliomas and account for 40-50% of brain tumors. They are common intracranial malignancies.

The α-amino acid-like boron trifluoride compound has a structure shown as formula (I):

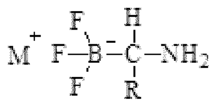

( I )

Wherein: R represents hydrogen, methyl, isopropyl, 1-methylpropyl, 2-methylpropyl, hydroxymethyl, 1-hydroxyethyl, benzyl or hydroxybenzyl.

The compound according to formula (I) can be obtained by the following preparation method, and the preparation route is as follows:

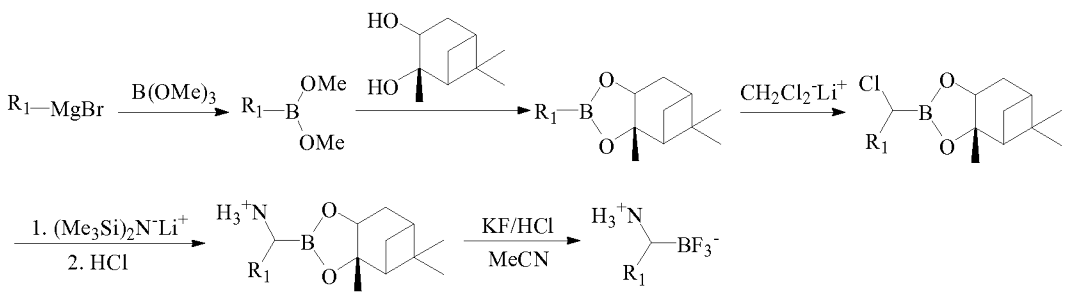


## DESCRIPTION OF THE FIGURES

FIG. 1 is a schematic plan view of an accelerator-based boron neutron capture therapy system.

FIG. 2 is a schematic plan view of a reactor-based boron neutron capture therapy system.

## DESCRIPTION OF THE DISCLOSURE

The following further describes the present disclosure in detail with reference to specific embodiments and the accompanying drawings so that those skilled in the art can implement the present disclosure with reference to the context of the specification. It will be understood that terms such as "having," "including," and "comprising" used herein do not exclude the presence or addition of one or more other components or combinations thereof.

The fast neutrons described herein are neutrons with an energy region greater than 40keV, the epithermal neutron energy region is between 0.5eV and 40keV, and the thermal neutron energy region is less than 0.5eV.

Neutron capture therapy (NCT) has been increasingly practiced as an effective cancer curing means in recent years, and BNCT is the most common. Neutrons for NCT may be supplied by nuclear reactors or accelerators. Take AB-BNCT for example, its principal components include, in general, an accelerator for accelerating charged particles (such as protons and deuterons), a target, a heat removal system and a beam shaping assembly. The accelerated charged particles interact with the metal target to produce the neutrons, and suitable nuclear reactions are always determined according to such characteristics as desired neutron yield and energy, available accelerated charged particle energy and current and materialization of the metal target, among which the most discussed two are $^{7}Li$ (p, n) $^{7}Be$ and $^{9}Be$ (p, n) $^{9}B$ and both are endothermic reaction. Their energy thresholds are 1.881MeV and 2.055MeV respectively. Epithermal neutrons at a keV energy level are considered ideal neutron sources for BNCT. Theoretically, bombardment

with lithium target using protons with energy slightly higher than the thresholds may produce neutrons relatively low in energy, so the neutrons may be used clinically without many moderations. However, Li (lithium) and Be (beryllium) and protons of threshold energy exhibit not high action cross section. In order to produce sufficient neutron fluxes, high-energy protons are usually selected to trigger the nuclear reactions.

The target, considered perfect, is supposed to have the advantages of high neutron yield, a produced neutron energy distribution near the epithermal neutron energy range (see details thereinafter), little strong-penetration radiation, safety, low cost, easy accessibility, high temperature resistance etc. But in reality, no nuclear reactions may satisfy all requests. The target in these embodiments of the present disclosure is made of lithium. However, well known by those skilled in the art, the target materials may be made of other metals besides the above-mentioned.

Requirements for the heat removal system differ as the selected nuclear reactions. $^{7}Li$ (p, n) $^{7}Be$ asks for more than $^{9}Be$ (p, n) $^{9}B$ does because of low melting point and poor thermal conductivity coefficient of the metal (lithium) target. In these embodiments of the present disclosure is $^{7}Li$ (p, n) $^{7}Be$.

No matter BNCT neutron sources are from the nuclear reactor or the nuclear reactions between the accelerator charged particles and the target, only mixed radiation fields are produced, that is, beams include neutrons and photons having energies from low to high. As for BNCT in the depth of tumors, except the epithermal neutrons, the more the residual quantity of radiation ray is, the higher the proportion of nonselective dose deposition in the normal tissue is. Therefore, radiation causing unnecessary dose should be lowered down as much as possible. Besides air beam quality factors, dose is calculated using a human head tissue prosthesis in order to understand dose distribution of the neutrons in the human body. The prosthesis beam quality factors are later used as design reference to the neutron beams, which is elaborated hereinafter.

The International Atomic Energy Agency (IAEA) has given five suggestions on the air beam quality factors for the clinical BNCT neutron sources. The suggestions may be used for differentiating the neutron sources and as reference for selecting neutron production pathways and designing the beam shaping assembly, and are shown as follows:

Epithermal neutron flux > $1 \times 10^{9}$ n/c$^{m2}$s

Fast neutron contamination < $2 \times 10^{-13}$ Gy-cm$^{2}$/n

Photon contamination < $2 \times 10^{-13}$ Gy-cm$^{2}$/n

Thermal to epithermal neutron flux ratio < 0.05

Epithermal neutron current to flux ratio > 0.7

Note: the epithermal neutron energy range is between 0.5eV and 40keV, the thermal neutron energy range is lower than 0.5eV, and the fast neutron energy range is higher than 40keV.

1. Epithermal neutron flux

The epithermal neutron flux and the concentration of the boronated pharmaceuticals at the tumor site codetermine clinical therapy time. If the boronated pharmaceuticals at the tumor site are high enough in concentration, the epithermal neutron flux may be reduced. On the contrary, if the concentration of the boronated pharmaceuticals in the tumors is at a low level, it is required that the epithermal neutrons in the high epithermal neutron flux should provide enough doses to the tumors. The given standard on the epithermal neutron flux from IAEA is more than 109 epithermal neutrons per square centimeter per second. In this flux of neutron beams, therapy time may be approximately controlled shorter than an hour with the boronated pharmaceuticals. Thus, except that patients are well positioned and feel more comfortable in shorter therapy time, and limited residence time of the boronated pharmaceuticals in the tumors may be effectively utilized.

2. Fast neutron contamination

Unnecessary dose on the normal tissue produced by fast neutrons are considered as contamination. The dose exhibit positive correlation to neutron energy, hence, the quantity of the fast neutrons in the neutron beams should be reduced to the greatest extent. Dose of the fast neutrons per unit epithermal neutron flux is defined as the fast neutron contamination, and according to IAEA, it is supposed to be less than $2*10^{-13}$Gy-cm$^{2}$/n.

3. Photon contamination (gamma-ray contamination)

Gamma-ray long-range penetration radiation will selectively result in dose deposit of all tissues in beam paths, so that lowering the quantity of gamma-ray is also the exclusive requirement in neutron beam design. Gamma-ray dose accompanied per unit epithermal neutron flux is defined as gamma-ray contamination which is suggested being less than $2*10^{-13}$Gy-$cm^2$/n according to IAEA.

4. Thermal to epithermal neutron flux ratio

The thermal neutrons are so fast in rate of decay and poor in penetration that they leave most of energy in skin tissue after entering the body. Except for skin tumors like melanocytoma, the thermal neutrons serve as neutron sources of BNCT, in other cases like brain tumors, the quantity of the thermal neutrons has to be lowered. The thermal to epithermal neutron flux ratio is recommended at lower than 0.05 in accordance with IAEA.

5. Epithermal neutron current to flux ratio

The epithermal neutron current to flux ratio stands for beam direction, the higher the ratio is, the better the forward direction of the neutron beams is, and the neutron beams in the better forward direction may reduce dose surrounding the normal tissue resulted from neutron scattering. In addition, treatable depth as well as positioning posture is improved. The epithermal neutron current to flux ratio is better of larger than 0.7 according to IAEA.

The prosthesis beam quality factors are deduced by virtue of the dose distribution in the tissue obtained by the prosthesis according to a dose-depth curve of the normal tissue and the tumors. The three parameters as follows may be used for comparing different neutron beam therapy effects.

1. Advantage depth

Tumor dose is equal to the depth of the maximum dose of the normal tissue. Dose of the tumor cells at a position behind the depth is less than the maximum dose of the normal tissue, that is, boron neutron capture loses its advantages. The advantage depth indicates penetrability of neutron beams. Calculated in cm, the larger the advantage depth is, the larger the treatable tumor depth is.

2. Advantage depth dose rate

The advantage depth dose rate is the tumor dose rate of the advantage depth and also equal to the maximum dose rate of the normal tissue. It may have effects on length of the therapy time as the total dose on the normal tissue is a factor capable of influencing the total dose given to the tumors. The higher it is, the shorter the irradiation time for giving a certain dose on the tumors is, calculated by cGy/mA-min.

3. Advantage ratio

The average dose ratio received by the tumors and the normal tissue from the brain surface to the advantage depth is called as advantage ratio. The average ratio may be calculated using dose-depth curvilinear integral. The higher the advantage ratio is, the better the therapy effect of the neutron beams is.

To provide comparison reference to design of the beam shaping assembly, we also provide the following parameters for evaluating expression advantages and disadvantages of the neutron beams in the embodiments of the present disclosure except the air beam quality factors of IAEA and the abovementioned parameters.

1. Irradiation time ≤30min (proton current for accelerator is 10mA)

2. 30.0RBE-Gy treatable depth≥7cm

3. The maximum tumor dose≥60.0RBE-Gy

4. The maximum dose of normal brain tissue ≤12.5RBE-Gy

5. The maximum skin dose ≤1 1.0RBE-Gy

Note: RBE stands for relative biological effectiveness. Since photons and neutrons express different biological effectiveness, the dose above should be multiplied with RBE of different tissues to obtain equivalent dose.

Please refer to FIG. 1, which shows a schematic plan view of an accelerator-based boron neutron capture therapy system, the boron neutron capture therapy system includes an accelerator 10, a beam expander 20, a charged particle beam inlet for passing the charged particle beam P, a charged particle beam P, a neutron generator T for generating a neutron beam N by nuclear reaction with the charged particle beam P, a beam shaping assembly 30 for adjusting the neutron beam flux and quality generated by the neutron generator T, a collimator 40 adjacent to the beam shaping assembly 30, and an $\alpha$-amino acid-like boron trifluoride compound 50 irradiated by a beam emerging from the collimator 40. Wherein, the accelerator 10 is used to accelerate the charged particle beam P and may be an accelerator suitable for an accelerator type neutron capture therapy system such as a cyclotron or a linear accelerator; the charged particle beam P herein is preferably a proton beam; the beam expander 20 is disposed between the accelerator 10 and the neutron generator T; the charged particle beam inlet is adjacent to the neutron generator T and accommodated in the beam shaping assembly 30, and the three arrows between the neutron generator T and the beam expander 20 serve as charged particle beam inlet; the neutron generator T is accommodated in the beam shaping assembly 30, and the neutron generator T herein is preferably lithium. The beam shaping assembly 30 includes a reflector 31, a moderator 32 surrounded by the reflector 31 and adjacent to the neutron generator T, a thermal neutron absorber 33 adjacent to the moderator 32, and a radiation shield 34 arranged in the beam shaping assembly 30. The neutron generator T generates the neutron beam N by a nuclear reaction with the charged particle beam P incident from the charged particle beam inlet, the moderator 32 moderates neutrons generated from the neutron generator T to an epithermal neutron energy region, the reflector 31 directs the deviated neutrons back to increase the intensity of the epithermal neutron beam, the thermal neutron absorber 33 is used to absorb thermal neutrons to avoid overdosing in superficial normal tissues during therapy, and the radiation shield 34 is used to shield leaking neutrons and photons to reduce dose of the normal tissue not exposed to irradiation. A collimator 40 is used to converge the neutron beam; and the energy generated from the action of the neutron beam emitted through the collimator 40 on the $\alpha$-amino acid-like boron trifluoride compound 50 destroys tumor cell DNA.

Please refer to FIG. 2 , which shows a schematic plan view of a reactor-based boron neutron capture therapy system, the boron neutron capture therapy system includes a reactor 100 (a neutron beam is generated in the reactor, so it can also be called neutron generator), beam expander 200, a neutron beam inlet, a beam shaping assembly 300 for adjusting the neutron beam flux and quality generated by the neutron generator, a collimator 400 adjacent to beam shaping assembly 300, and an $\alpha$-amino acid-like boron trifluoride compound 500 irradiated by a beam emerging from the collimator 40. Wherein, the reactor 100 may be a related nuclear reaction capable of generating neutron with required energy, which is well known to those skilled in the art, such as fast neutrons generated from fission reactions of uranium-235 or plutonium-239; the beam expander 200 is disposed between the reactor 100 and the neutron beam inlet; and the three arrows following the beam expander 200 serve as neutron beam inlets. The beam shaping assembly 300 includes a reflector 310, a moderator 320 surrounded by the reflector 310, a thermal neutron absorber 330 adjacent to the moderator 320, and a radiation shield 340 arranged in the beam shaping assembly 300. The moderator 32 moderates neutrons generated from the neutron generator 100 to an epithermal neutron energy zone, the reflector 310 directs the deviated neutrons back to increase the intensity of the epithermal neutron beam, the thermal neutron absorber 330 is used to absorb thermal neutrons to avoid overdosing in superficial normal tissues during therapy, and the radiation shield 340 is used to shield leaking neutrons and photons to reduce dose of the normal tissue not exposed to irradiation. The collimator 400 is used to converge the neutron beam; and the energy generated from the action of the neutron beam emitted through the collimator 400 on the $\alpha$-amino acid-like boron trifluoride compound 500 destroys tumor cell DNA.

The beam shaping assembly 30, 300 can moderate the neutrons to the epithermal neutron energy region and reduce the thermal neutron and fast neutron content. The reflectors 31, 310 are made of a material having strong neutron reflection capability. As a preferred embodiment, the reflectors 31, 310 are made of at least one of Pb or Ni. The moderators 32, 320 are made of a material having a large action cross section for fast neutron and a small action cross section for epithermal neutron. As a preferred embodiment, the moderators 32, 320 are made of at least one of $D_2O$, $AlF_3$, Fluental™, $CaF_2$, $Li_2CO_3$, $MgF_2$ and $Al_2O_3$. The thermal neutron absorbers 33, 330 are made of a material having a large action cross section for thermal neutron. As a preferred embodiment, the thermal neutron absorbers 33, 330 are made of $^6Li$. The radiation shields 34, 340 include photon shield and neutron shield. As a preferred embodiment, the radiation shields 34, 340 include a photon shield made of Pb and a neutron shield made of polyethylene (PE). The collimators 40, 400 are made of a material having a strong neutron gathering capability. As a preferred embodiment, the collimators 40, 400 are made of at least one of graphite and Pb.

Those skilled in the art know that, besides the above accelerator type and reactor type neutron generation methods, other neutron generation methods, such as D-D neutron generator, D-T neutron generator, etc., can also be used according to actual needs. The material, structure, and composition of the beam shaping assembly can also be adjusted according to actual needs.

In BNCT, when administered in a therapeutically effective amount, the boron-containing compound must be non-toxic or low toxic, and can selectively accumulate in tumor tissue. Although BPA has the advantage of low chemical

toxicity, it accumulates in critical normal tissues at below-desired levels. In particular, the ratio of boron in tumor relative to normal brain and tumor to blood is approximately 3:1. This low specificity limits the maximum dose of BPA to the tumor because the allowable dose for normal tissue is a limiting factor.

Therefore, there is a need to develop new compounds that have a longer retention time in tumors and selectively target and destroy tumor cells with minimal damage to normal tissues. α-Amino acids are the main components of proteins and are the most important amino acids in organisms. They play a very important role in the production of ATP and in the process of neurotransmission. In addition, α-amino acids are also key nutrients for the survival and proliferation of cancer cells. α-Amino acid-like boron trifluoride compound are obtained by replacing -COOH in the α-amino acid with -$BF_3$, which is an α-amino acid isoelectronic compound. Studies have shown that cellular pathways for ingesting α-amino acid-like boron trifluoride compounds are the same as α-amino acids, and they all adopt enzyme-mediated pathways, and both have the same transporter. The α-amino acid-like boron trifluoride compounds have attracted our intense attention in the design of novel boron carrier compounds for BNCT, which have high stability, good targeting, and high enrichment in tumor cells. Compared to FDG, the uptake of these compounds by the inflammatory region is almost negligible. In addition, the α-amino acid-like boron trifluoride compounds are readily synthesized and are usually prepared by reacting the corresponding boronic acid ester with $KHF_2$ under acidic conditions.

In addition, using $^{18}F$-labeled α-amino acid-like boron trifluoride compounds in BNCT, boron concentrations and distributions in and around tumors and all tissues within the radiation therapy volume can be measured non-invasively and accurately before and during irradiation. This diagnostic information enables boron neutron capture therapy to be performed faster, more accurately, and more safely by reducing the exposure of epithermal neutrons to tissue regions known to contain high levels of boron.

The α-amino acid-like boron trifluoride compound is described in details below in connection with specific examples.

<u>Example 1</u> Preparation of Phe-$BF_3$

Preparation route:

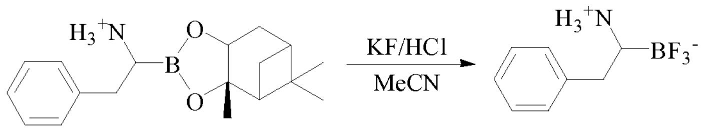


Benzyl borate (15 mg, 0.05 mmol), KF (0.15 mmol, 0.05 mL) solution, HCl (0.2 mmol, 0.03 mL) solution, 0.1 mL MeCN solution were added to a 1.5 mL microreactor and reacted for 2 h at room temperature to give crude Phe-$BF_3$. The crude product was further purified by HPLC to give Phe-$BF_3$. $^1H$ NMR (300 MHz, MeOD): δppm 7.30 (m, 5H), 3.04 (d,J=9.8Hz, 1H), 2.67 (t, J=9.8Hz, 1H), 2.42 (brs, 1H); $[M-H]^-$ 188.0901, Found: 188.05890.

*In vitro* study of the compound according to the disclosure

An *in vitro* test of the Phe-$BF_3$ of the Example 1 uses four different human-derived tumor cell lines U343mga, human liver cancer cell line Hep3B, human breast cancer cell line MCF7 and human Sarcoma cell line 4SS. Cells were plated on uncoated tissue culture dishes and incubated at 37°C in an incubator with humidified air equilibrated with 5% $CO_2$ (the medium was supplemented with 10% FCS and PEST (Penicillin 100 IU/mL and streptomycin 100 mg/mL)). For cell passage, cells were trypsinized with trypsin-EDTA (phosphate buffered saline (PBS) with 0.25% trypsin and 0.02% EDTA, no calcium and magnesium).

<u>Example 2</u> Cell uptake of Phe-$BF_3$

U343mga cells were plated on Petri dishes at a cell density of 75% and incubated for 6 hours with 1,4-dihydroxyborylphenylalanine (BPA) or Phe-$BF_3$ dissolved in tissue culture medium. Both boron-containing compounds were added at an equimolar concentration relative to the boron content ($5\times10^{-4}$mol/L boron) and dissolved in tissue culture medium. Incubation was terminated by removing the boron containing tissue culture medium and adding cold phosphate buffered saline solution (PBS buffer) in order to wash away excess medium from the cells. Cells were immediately harvested by scooping off from the petri dishes using rubber policeman, and collected in cold PBS and pelleted by centrifugation.

Total protein analysis was performed on cell samples according to the Bradford standard procedure. The precipitated cells were subjected to boron analysis by DC-plasmon atomic emission spectroscopy (DCP-AES). The sample (50-130 mg) was digested with sulfuric acid/nitric acid (1/1) at 60°C. Triton X-100 and water were added to give concentrations of 50 mg tissue/mL, 15% total acid v/v, and 5% Triton X-100 v/v. The boron concentration is based on known control sample. The results are shown in Table 1 below. As can be seen from Table 1, Phe-$BF_3$ is better than boron phenylalanine (BPA) in boron uptake.

Table 1: Cell uptake of different boron compounds

For the different boron compounds in the two parallel experiments (Trials 1 and 2), the boron content is expressed as a function of the total cellular protein in U343mga cells (7.2 and 7.7 μg boron/mL media in Trial 1 and Trial 2, respectively).

| Boron compound | Trial 1 | Trial 2 |
|---|---|---|
| BPA | 62 | 39 |
| Phe-$BF_3$ | 549 | 421 |

Example 3 Uptake of Phe-$BF_3$ by different tumor cells

Four human-derived, different tumor cell lines: U343mga, Hep3B, MCF7, and 4SS were plated on Petri dishes at 40-50% (low) and 90-100% (high) cell densities, and incubated with Phe-$BF_3$ in tissue culture as described above for 6 hours. Incubation was terminated by removing the boron-containing media and adding cold PBS buffer to wash excess media from the cells. Cells were immediately harvested by scooping off from the petri dishes using rubber policeman, and collected in cold PBS and pelleted by centrifugation. Total protein analysis was performed on cell samples according to the Bradford standard procedure (as described above). The results are shown in Table 2 below. From a comparison of all four human tumor cell lines tested at low and high cell densities, Phe-$BF_3$ was found to be a highly efficient boron carrier.

Table 2: Cell uptake of Phe-$BF_3$. The boron content is expressed as a function of the total cellular protein (μg boron/g cell protein).

| Cell line | Low cell density | High cell density |
|---|---|---|
| U343mga | 102 | 178 |
| Hep3B | 149 | 207 |
| MCF7 | 101 | 167 |
| 4SS | 255 | 343 |

Example 4 Intracellular retention of Phe-$BF_3$

U343mga cells were plated on Petri dishes at a cell density of 75% and incubated for 18 hours with 1,4-dihydroxyboron-phenylalanine (BPA) or Phe-$BF_3$ in tissue culture medium. Both boron compounds were added to the tissue culture medium at equimolar concentrations relative to the boron content ($5\times10^{-4}$mol/L boron). The incubation was terminated by replacing the boron-containing medium with a boron-free medium. Cell samples were taken at time points 0, 2 and 7 hours, respectively, where the 0 time point represented the time point when the incubation with the boron compound reached just 18 hours.

The cells were washed with cold PBS and immediately harvested by scooping off from the petri dish using rubber policeman, and collected in cold PBS and pelleted by centrifugation. The cell pellets were analyzed for total protein and boron content as described above. The results are shown in Table 3 below. With intracellular uptake, the compound of formula (I) retained in the tumor cells was 50% of the total uptake at 7 h after the complete consumption of Ia in the medium.

Table 3: Boron content (μg boron/g cell pellet) in U343mga cells at 0, 2 and 7 h after removal of the boron-containing medium.

| Boron compound | 0h | 2h | 7h |
|---|---|---|---|
| BPA | 0.072 | 0 | 0 |
| Ia | 0.83 | 0.56 | 0.41 |

In summary, as shown in Examples 2-4, the compound Phe-$BF_3$ has shown the expected results in an *in vitro* assay, which is superior to BPA in tumor cell uptake, accumulation, and retention.

Example 5 Phe-$BF_3$ cytotoxicity study test

The cell culture fluid containing the peptide bovine serum was incubated at 37C for 24 hours. The passage cultured mouse fibroblast L-929 cells were used to prepare a cell suspension of $1\times10^5$ cells/mL, and the cell suspension was seeded on a 96-well cell culture plate (100 μl/well) and cultured in a carbon dioxide incubator at 37°C for 24 hours. After the cells adhere to the wall, the supernatant was removed, the control solution (in absence of compound Ia) was added, and the culture solution of the test group (Phe-$BF_3$ concentration of 5 mmol/L) was exchanged, and the culture was continued in the carbon dioxide incubator at 37°C. After 2 days, MTT solution was added to continue culture for 4 hours. The original solution was aspirated and DMSO was added and shaken for 10 min. The absorbance value was measured with an enzyme-linked immunometric instrument at a wavelength of 630 nm, and the relative proliferation rate (RGR) of the cells was calculated according to the absorbance according to the formula. The results are shown in Table 4 below.

Table 4: Relative cell proliferation (RGR) results measured by MTT colorimetry

| NO. | Time (days) | RGR (%) |
|---|---|---|
| 1 | 2 | 123 |
| 2 | 7 | 102 |

$$\text{Note: RGR (\%)} = \frac{\text{average absorbance of test group}}{\text{average absorbance of control group}} \times 100\%.$$

Cellular toxicity was assessed based on cell relative proliferation, shown in Table 5 below.

Table 5: Assessment of cytotoxicity

| Grade | Relative proliferation RGR (%) |
|---|---|
| 0 | ≥ 100 |
| 1 | 75 ~ 99 |
| 2 | 50 ~ 74 |
| 3 | 25 ~ 49 |
| 4 | 1 ~ 24 |
| 5 | 0 |

As can be seen from Table 5, Phe-$BF_3$ showed no signs of toxicity.

## Claims

**1.** A boron neutron capture therapy system comprising:

a boron neutron capture therapy device; and
an α-amino acid-like boron trifluoride compound having a structure shown as formula (I) below:

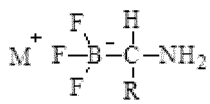

(I)

wherein, R represents hydrogen, methyl, isopropyl, 1-methylpropyl, 2-methylpropyl, hydroxymethyl, 1-hydroxyethyl, benzyl or hydroxybenzyl;

M represents H or metal atom;
the energy generated from the action of the neutron beam generated by the boron neutron capture therapy device on the α-amino acid-like boron trifluoride compound destroys tumor cell DNA.

**2.** The boron neutron capture therapy system according to claim 1, wherein the boron neutron capture therapy device comprises a neutron generator and a beam shaping assembly for adjusting the neutron beam energy spectrum generated by the neutron generator to an epithermal neutron energy zone.

**3.** The boron neutron capture therapy system according to claim 2, wherein the neutron generator generates neutrons by a nuclear reaction with the incident proton beam, the beam shaping assembly comprises:

a moderator adjacent to the neutron generator, wherein the moderator moderates neutrons generated from the neutron generator to an epithermal neutron energy zone,
a reflector surrounding the moderator, wherein the reflector leads the deflected neutrons back to increase the intensity of an epithermal neutron beam,
a thermal neutron absorber adjacent to the moderator, wherein the thermal neutron absorber absorbs thermal neutrons to avoid overdosing in superficial normal tissues during therapy, and
a radiation shield arranged in the beam shaping assembly, wherein the radiation shield shields leaking neutrons and photons to reduce dose of the normal tissue not exposed to irradiation.

**4.** The boron neutron capture therapy system according to claim 3, wherein the boron neutron capture therapy device further comprises a collimator disposed at a beam outlet for converging the epithermal neutrons.

**5.** The boron neutron capture therapy system according to any one of claims 1-4, wherein M represents potassium or sodium.

**6.** The boron neutron capture therapy system according to any one of claims 1-4, wherein B is $^{10}B$.

**7.** The boron neutron capture therapy system according to claim 6, wherein the purity of $^{10}B$ in the α-amino acid-like boron trifluoride compound is ≥ 95%.

**8.** The boron neutron capture therapy system according to any one of claims 1-4, wherein at least one F atom of the α-amino acid-like boron trifluoride compound is $^{18}F$.

**9.** An α-amino acid-like boron trifluoride compound having a structure shown as formula (I) below:

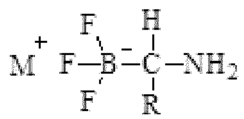

( I )

wherein, R represents hydrogen, methyl, isopropyl, 1-methylpropyl, 2-methylpropyl, hydroxymethyl, 1-hydroxyethyl, benzyl or hydroxybenzyl;
M represents H or metal atom

for use in tumor therapy.

**10.** The α-amino acid-like boron trifluoride compound for use according to claim 9, wherein M represents potassium or sodium.

**11.** The α-amino acid-like boron trifluoride compound for use according to any one of claims 9-10, wherein the tumor therapy refers to boron neutron capture therapy of the tumor.

**12.** The α-amino acid-like boron trifluoride compound for use according to claim 11, wherein the tumor is a malignant tumor or metastatic tumor.

**13.** The α-amino acid-like boron trifluoride compound for use according to claim 11, wherein the tumor is glioma, recurrent

head and neck tumor, malignant melanoma, breast cancer or metastatic liver cancer.

**14.** The α-amino acid-like boron trifluoride compound for use according to claim 13, wherein the tumor is glioma or malignant melanoma.

## Patentansprüche

**1.** Borneutroneneinfang-Therapiesystem umfassend:

eine Borneutroneneinfang-Therapievorrichtung; und
eine α-Aminosäure ähnliche Bortrifluoridverbindung, die eine Struktur aufweist, die als Formel (I) unten gezeigt ist:

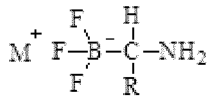

(I)

wobei R Wasserstoff, Methyl, Isopropyl, 1-Methylpropyl, 2-Methylpropyl, Hydroxymethyl, 1-Hydroxymethyl, Benzyl oder Hydroxybenzyl darstellt;
M H oder ein Metallatom darstellt;

die Energie, die durch die Wirkung des Neutronenstrahls, der durch die Borneutroneneinfang-Therapievorrichtung erzeugt wird, auf die α-Aminosäure ähnliche Bortrifluoridverbindung erzeugt wird, die Tumorzellen-DNA zerstört.

**2.** Borneutroneneinfang-Therapiesystem nach Anspruch 1, wobei die Borneutroneneinfang-Therapievorrichtung einen Neutronengenerator und eine Strahlformungsanordnung zum Einstellen des Neutronenstrahlenergiespektrums, das durch den Neutronengenerator erzeugt wird, auf eine epithermale Neutronenenergiezone umfasst.

**3.** Borneutroneneinfang-Therapiesystem nach Anspruch 2, wobei der Neutronengenerator Neutronen durch eine Kernreaktion mit dem einfallenden Protonenstrahl erzeugt, wobei die Strahlformungsanordnung Folgendes umfasst:

einen Moderator, der dem Neutronengenerator benachbart ist, wobei der Moderator Neutronen, die aus dem Neutronengenerator erzeugt werden, zu einer epithermalen Neutronenenergiezone moderiert,
einen Reflektor, der den Moderator umgibt, wobei der Reflektor die abgelenkten Neutronen zurückführt, um die Intensität eines epithermalen Neutronenstrahls zu erhöhen,
einen thermischen Neutronenabsorber, der dem Moderator benachbart ist, wobei der thermische Neutronenabsorber thermische Neutronen absorbiert, um das Überdosieren normaler Oberflächengewebe während der Therapie zu vermeiden und
eine Strahlungsabschirmung, die in der Strahlformungsanordnung angeordnet ist, wobei die Strahlungsabschirmung entweichende Neutronen und Photonen abschirmt, um die Dosis für das normale Gewebe, das der Strahlung nicht ausgesetzt wird, zu reduzieren.

**4.** Borneutroneneinfang-Therapiesystem nach Anspruch 3, wobei die Borneutroneneinfang-Therapievorrichtung einen Kollimator umfasst, der an einem Strahlauslass zum Zusammenführen der epithermalen Neutronen angeordnet ist.

**5.** Borneutroneneinfang-Therapiesystem nach irgendeinem der Ansprüche 1-4, wobei M Kalium oder Natrium darstellt.

**6.** Borneutroneneinfang-Therapiesystem nach irgendeinem der Ansprüche 1-4, wobei B $^{10}B$ ist.

**7.** Borneutroneneinfang-Therapiesystem nach Anspruch 6, wobei die Reinheit von $^{10}B$ in der α-Aminosäure ähnlichen Bortrifluoridverbindung ≥ 95 % beträgt.

**8.** Borneutroneneinfang-Therapiesystem nach irgendeinem der Ansprüche 1-4, wobei mindestens ein F-Atom der α-Aminosäure ähnlichen Bortrifluoridverbindung $^{18}F$ ist.

**9.** α-Aminosäure ähnliche Bortrifluoridverbindung, die eine Struktur aufweist, die unten als Formel (I) gezeigt ist,

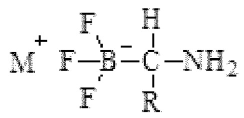

(I)

wobei R Wasserstoff, Methyl, Isopropyl, 1-Methylpropyl, 2-Methylpropyl, Hydroxymethyl, 1-Hydroxymethyl, Benzyl oder Hydroxybenzyl darstellt;
M H oder ein Metallatom darstellt,

zur Verwendung bei der Tumortherapie.

**10.** α-Aminosäure ähnliche Bortrifluoridverbindung zur Verwendung nach Anspruch 9, wobei M Kalium oder Natrium darstellt.

**11.** α-Aminosäure ähnliche Bortrifluoridverbindung zur Verwendung nach irgendeinem der Ansprüche 9-10, wobei die Tumortherapie sich auf Borneutroneneinfang-Therapie des Tumors bezieht.

**12.** α-Aminosäure ähnliche Bortrifluoridverbindung nach Anspruch 11, wobei der Tumor ein bösartiger Tumor oder ein metastatischer Tumor ist.

**13.** α-Aminosäure ähnliche Bortrifluoridverbindung zur Verwendung nach Anspruch 11, wobei der Tumor Gliom, wiederholt auftretender Kopf- und Halstumor, bösartiges Melanom, Brustkrebs oder metastatischer Leberkrebs ist.

**14.** α-Aminosäure ähnliche Bortrifluoridverbindung zur Verwendung nach Anspruch 13, wobei der Tumor Gliom oder bösartiges Melanom ist.

## Revendications

**1.** Système de thérapie de capture de neutrons par le bore comprenant :

un dispositif de thérapie de capture de neutrons par le bore; et
composé de trifluorure de bore ressemblant à un α-acide aminé ayant une structure répondant à la formule (I) ci-dessous :

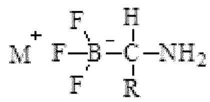

(I)

dans laquelle R représente un atome d'hydrogène, un groupe méthyle, isopropyle, 1-méthylpropyle, 2-méthylpropyle, hydroxyméthyle, hydroxyéthyle, benzyle ou hydroxy benzyle ;
M représente H ou un atome de métal ;

l'énergie générée par l'action du faisceau de neutrons généré par le dispositif de thérapie de capture de neutrons par le bore sur le composé de trifluorure de bore ressemblant à un α-acide aminé détruit l'ADN cellulaire tumoral.

**2.** Système de thérapie de capture de neutrons par le bore selon la revendication 1, dans lequel le dispositif de thérapie de capture de neutrons par le bore comprend un générateur de neutrons et un ensemble de mise en forme de faisceau permettant de régler le spectre d'énergie de faisceau de neutrons généré au moyen du générateur de neutrons sur une zone d'énergie de neutrons épithermiques.

**3.** Système de thérapie de capture de neutrons par le bore selon la revendication 2, dans lequel le générateur de

neutrons génère des électrons au moyen d'une réaction nucléaire avec le faisceau de protons incidents, l'ensemble de mise en forme de faisceau comprend :un modérateur adjacent au générateur de neutrons, dans lequel le modérateur modère des neutrons générés à partir du générateur de neutrons sur une zone d'énergie de neutrons épithermiques,un réflecteur entourant le modérateur, dans lequel le réflecteur ramène les neutrons déviés de manière à augmenter l'intensité d'un faisceau de neutrons épithermiques,
un absorbeur de neutrons thermiques adjacent au modérateur, dans lequel l'absorbeur de neutrons thermiques absorbe les neutrons thermiques afin d'éviter un surdosage dans les tissus normaux superficiels pendant la thérapie, et
un écran de protection radiologique agencé dans l'ensemble de mise en forme de faisceau, dans lequel l'écran de protection radiologique protège contre des neutrons et des photons s'échappant afin de réduire la dose du tissu normal non exposé à un rayonnement.

4. Système de thérapie de capture de neutrons par le bore selon la revendication 3, dans lequel le dispositif de thérapie de capture de neutrons par le bore comprend en outre un collimateur disposé au niveau d'une sortie de faisceau permettant de faire converger les neutrons épithermiques.

5. Système de thérapie de capture de neutrons par le bore selon l'une quelconque des revendications 1 à 4, dans lequel M représente le potassium ou le sodium.

6. Système de thérapie de capture de neutrons par le bore selon l'une quelconque des revendications 1 à 4, dans lequel B représente le $^{10}B$.

7. Système de thérapie de capture de neutrons par le bore selon la revendication 6, dans lequel la pureté de $^{10}B$ dans le composé de trifluorure de bore ressemblant à un $\alpha$-acide aminé est supérieure ou égale à 95 %.

8. Système de thérapie de capture de neutrons par le bore selon l'une quelconque des revendications 1 à 4, dans lequel au moins un atome de F dans le composé de trifluorure de bore ressemblant à un $\alpha$-acide aminé est le $^{18}F$.

9. Composé de trifluorure de bore ressemblant à un $\alpha$-acide aminé ayant une structure répondant à la formule (I) ci-dessous :

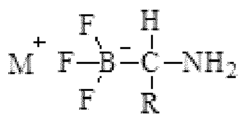

( I )

dans laquelle R représente un atome d'hydrogène, un groupe méthyle, isopropyle, 1-méthylpropyle, 2-méthylpropyle, hydroxyméthyle, hydroxyéthyle, benzyle ou hydroxy benzyle ;
M représente H ou un atome de métal ;

destiné à être utilisé en thérapie tumorale.

10. Composé de trifluorure de bore ressemblant à un $\alpha$-acide aminé destiné à être utilisé selon la revendication 9, dans lequel M représente le potassium ou le sodium.

11. Composé de trifluorure de bore ressemblant à un $\alpha$-acide aminé destiné à être utilisé selon l'une quelconque des revendications 9 ou 10, dans lequel la thérapie tumorale fait référence à la thérapie de capture de neutrons par le bore de la tumeur.

12. Composé de trifluorure de bore ressemblant à un $\alpha$-acide aminé destiné à être utilisé selon la revendication 11, dans lequel la tumeur est une tumeur maligne ou une tumeur métastatique.

13. Composé de trifluorure de bore ressemblant à un $\alpha$-acide aminé destiné à être utilisé selon la revendication 11, dans lequel la tumeur est un gliome, une tumeur récurrente de la tête et du cou, un mélanome maligne, le cancer du sein ou le cancer du foie métastatique.

**14.** Composé de trifluorure de bore ressemblant à un α-acide aminé destiné à être utilisé selon la revendication 13, dans lequel la tumeur est un gliome ou un mélanome maligne.

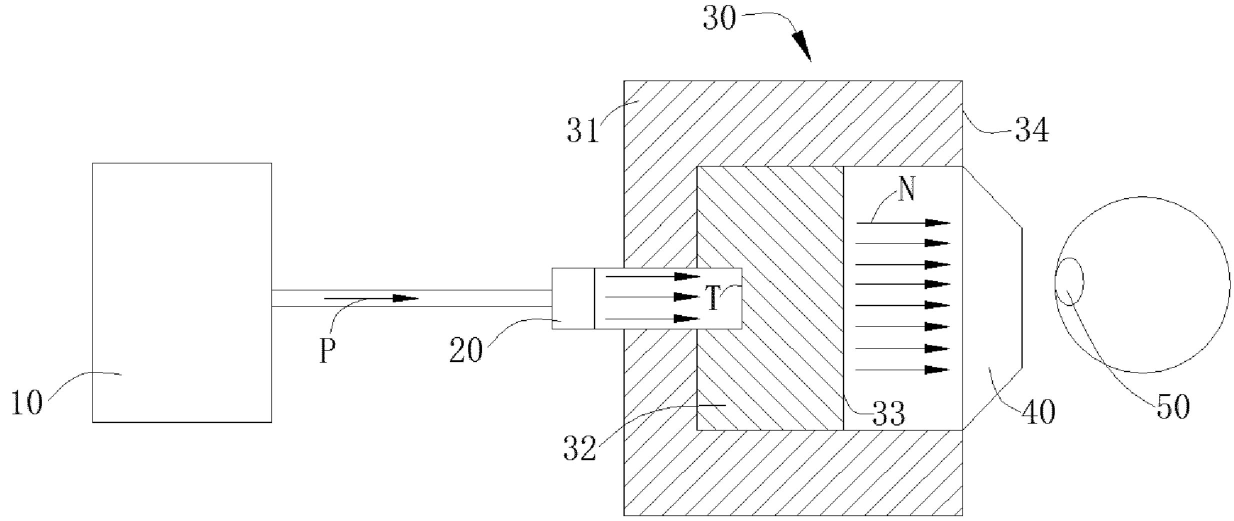


FIG. 1

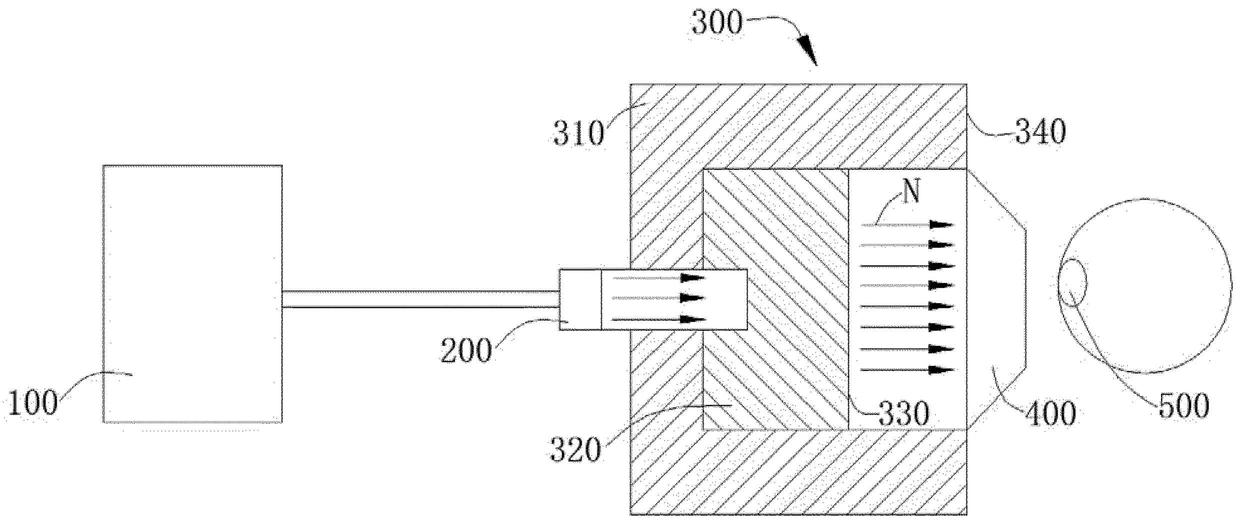


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014007831 A1

### Non-patent literature cited in the description

- **LIU Z. et al.** *Science Adv,* 2015, vol. 1 (8), el500694
- **BARTH R.F. et al.** *Clinical Cancer Research,* 2005, vol. 11 (11), 3987-4002